# EUROPEAN PATENT SPECIFICATION

(11) **EP 2 522 354 B1**
(45) Date of publication and mention of the grant of the patent: **23.08.2017**
(21) Application number: 11165869.6
(22) Date of filing: 12.05.2011
(51) Int. Cl.: A61K 31/727, A61K 35/16, A61K 38/57, A61P 7/02, A61P 1/00, A61P 11/08, A61P 9/06, A61P 9/12

(54) **Methods to reduce adverse events caused by pharmaceutical preparations comprising plasma derived proteins**
Verfahren zur Verringerung von durch pharmazeutische Präparate mit Plasma-abgeleiteten Proteinen verursachten unerwünschten Ereignissen
Procédé de réduction des événements indésirables causés par les préparations pharmaceutiques comportant des protéines dérivées du plasma

(43) Date of publication of application: 14.11.2012
(73) Proprietor: CSL Behring GmbH, 35041 Marburg (DE)
(72) Inventor: Schulte, Stefan, 35043 Marburg (DE); Kalina, Uwe, 35041 Marburg (DE); Moses, Michael, 61279 Grävenwiesbach (DE); Feussner, Annette, 35043 Marburg (DE)
(74) Representative: Hauser, Hans-Peter

(56) References cited:
- US-A- 4 388 232
- US-A1- 2006 035 828
- HOLMER E ET AL: "The molecular-weight dependence of the rate-enhancing effect of heparin on the inhibition of thrombin, factor Xa, factor IXa, factor XIa, factor XIIa and kallikrein by antithrombin", 1981, BIOCHEMICAL JOURNAL 1981 GB, VOL. 193, NR. 2, PAGE(S) 395 - 400, XP002661553, ISSN: 0264-6021 * See abstract and results: heparin potentiates the inhibiting activity of antithrombin (antithrombin III) on clotting factors, including kallikrein *
- OLSON S T ET AL: "High molecular weight kininogen potentiates the heparin-accelerated inhibition of plasma kallikrein by antithrombin: role for antithrombin in the regulation of kallikrein.", 16 November 1993 (1993-11-16), BIOCHEMISTRY 16 NOV 1993 LNKD- PUBMED:7692967, VOL. 32, NR. 45, PAGE(S) 12136 - 12147, XP002661554, ISSN: 0006-2960 * See abstract: heparin accelerates the inhibiting activity of antithrombin on kallikrenin *
- MACLENNAN ET AL: "Risks and side effects of therapy with plasma and plasma fractions", BEST PRACTICE & RESEARCH CLINICAL HAEMATOLOGY, BAILLIÈRE TINDALL, vol. 19, no. 1, 1 March 2006 (2006-03-01), pages 169-189, XP005219068, ISSN: 1521-6926

## Description

The instant invention provides a method to reduce the activity of FXIa in an immunological pharmaceutical preparation derived from a plasma fraction said plasma fraction comprising antithrombin III, the method comprising contacting the plasma fraction with heparin thereby reducing the activity of FXIa per ml of the plasma fraction, wherein the heparin is covalently coupled to a matrix and the plasma fraction has been preadsorbed to an anion exchange matrix, wherein the anion exchange matrix is an anion exchange membrane.

In the classical waterfall model, blood coagulation proceeds by a series of reactions involving the activation of zymogens by limited proteolysis culminating in the generation of thrombin, which converts plasma fibrinogen to fibrin and activates platelets. In turn, collagen- or fibrin-adherent platelets facilitate thrombin generation by several orders of magnitude via exposing procoagulant phospholipids (mainly phosphatidyl serine) on their outer surface, which propagates assembly and activation of coagulation protease complexes and by direct interaction between platelet receptors and coagulation factors.

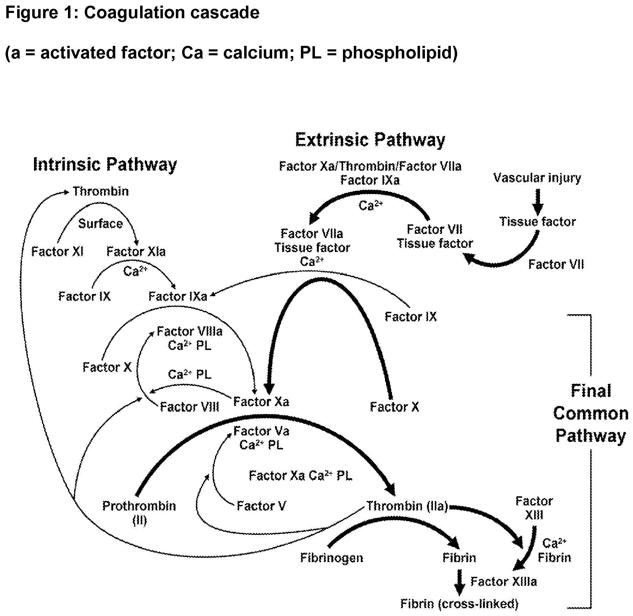

Two converging pathways for coagulation exist that are triggered by either extrinsic (vessel wall) or intrinsic (blood-borne) components of the vascular system (Figure 1). The "extrinsic" pathway is initiated by the complex of the serine protease factor VII (FVII) with the integral membrane protein tissue factor (TF), an essential coagulation cofactor that is absent on the luminal surface but strongly expressed in subendothelial layers of the vessel and which is accessible or liberated via tissue injury. TF expressed in circulating microvesicles might also contribute to thrombus propagation by sustaining thrombin generation on the surface of activated platelets.

The "intrinsic" or "contact activation pathway" is initiated when the serine protease factor XII (FXII, Hageman factor) comes into contact with negatively charged surfaces in a reaction involving high molecular weight kininogen and the serine protease plasma kallikrein (contact activation). FXII can be activated by macromolecular constituents of the subendothelial matrix such as glycosaminoglycans and collagens, sulfatides, nucleotides and other soluble polyanions or non-physiological material such as glass or polymers. One of the most potent contact activators is kaolin and this reaction serves as the mechanistic basis for the major clinical clotting test, the activated partial thromboplastin time (aPTT), which measures the coagulation function of the "intrinsic" pathway. In reactions propagated by platelets, activated FXII then activates the serine protease FXI to FXIa and subsequently FXIa activates the serine protease FIX to FIXa. The complex of FVIIIa, which FVIIIa has been previously activated by traces of FXa and/or thrombin, and FIXa (the tenase complex) subsequently activates the serine protease FX to FXa which in turn with FVa activates the serine protease prothrombin to thrombin.

Factor XIIa has a number of target proteins, including plasma prekallikrein and factor XI. Active plasma kallikrein further activates factor XII, leading to an amplification of contact activation. Contact activation is a surface mediated process responsible in part for the regulation of thrombosis and inflammation, and is mediated, at least in part, by fibrinolytic-, complement-, kininogen/kinin-, and other humoral and cellular pathways. The inactive precursor of plasma kallikrein, prekallikrein is synthesized in the liver as a one chain a-globulin with a molecular weight of approximately 88 kilodalton (kDa) [3]. Prekallikrein circulates in plasma as a 1:1 complex with HMWK in the concentration of 35-50 IlgjmL. The kallikrein is formed by the cleavage of prekallikrein into two chains which are held together by one disulfide bridge. The activation of prekallikrein to kallikrein is brought about by the active FXII (FXIIa). The active plasma kallikrein cleaves from the HMWK the biologically very active peptide bradykinin which produces heavy blood pressure decrease, increase of vessel permeability, release of tissue plasminogen activator (t-PA) and mobilization of arachidonic acid. Through these mechanisms the kallikrein-kinin-system influences regulation of the blood pressure, the function of kidney and heart as well as the pathological processes of inflammation (for review, Coleman, R. Contact Activation Pathway, pages 103-122 in Hemostasis and Thrombosis, Lippincott Williams Wilkins 2001; Schmaier A. H. Contact Activation, pages 105-128 in Thrombosis and Hemorrhage, 1998).

In pathological conditions, the coagulation cascade may be activated inappropriately which then results in the formation of hemostatically acting plugs inside the blood vessels. Thereby, vessels can be occluded and the blood supply to distal organs is limited. Furthermore, formed thrombin can detach and embolize into other parts of the body, there leading to ischemic occlusion. This process is known as thromboembolism and is associated with high mortality.

Activated proteases originating from blood plasma proteins may contaminate pharmaceutical preparations of proteins derived from human blood plasma and may be the cause of thromboembolic adverse events (TAEs). Suppliers of plasma derived pharmaceuticals therefore need to ensure that their products do not cause such TAEs which have also been associated with the use of an intravenous immunoglobulin (IVIG) preparation recently. Some authors attribute activated coagulation Factor XI (FXIa) with a relevant role in the thrombogenic potential of IVIGs (Alving BM, Tankersley DL, Mason BL, Rossi F, Aronson DL, Finlayson JS. Contact-activated factors: contaminants of immunoglobulins preparations with coagulant and vasoactive properties. J Lab Clin Med1980; 96(2): 334-46).

Apart from thrombotic events like stroke other adverse events may be caused by plasma protein preparation comprising enhanced concentrations of kallikrein FXIa or FXIIa such as skin reactions, bronchospasms, hypoxia, severe rigors, tachycardia, stomach aches and raised blood pressure.

The development of pure and safe preparations is a major goal of plasma derivative manufacturers, including diminishing or virtually eliminating the risk of IVIG-associated TAEs. Marzo et al. reported that pasteurization may be one means to reduce activated proteases in immunoglobulin preparations (Jose M, Marzo N, Bono M, Carretero M, Maite L, Ristol P, Jorquera J. Pasteurization Inactivates Clotting Enzymes During Flebogamma® And Flebogamma® Dif Production. WebmedCentral IMMUNOTHERAPY 2010;1(12): WMC001425).

In 2010 an i.v. immunoglobulin product was withdrawn due to thromboembolic events (European Medicines Agency. Questions and answers on the suspension of the marketing authorisations for Octagam (human normal immunoglobulin 5% and 10%). Outcome of a procedure under Article 107 of Directive 2001/83/EC. 23 September 2010) which led to the suspension of the marketing authorization of the respective product.

There is a clear medical need to develop alternative manufacturing methods which can be used to improve the safety of plasma derived pharmaceutical preparations.

The present invention provides a solution to this problem. In the method it was found that an adsorption of a pharmaceutical preparation or its intermediate fraction derived from plasma which comprises antithrombin III to heparin or heparin-like matrices can substantially reduce the amount of activated proteases and can thus considerably improve the safety of said pharmaceutical preparation.

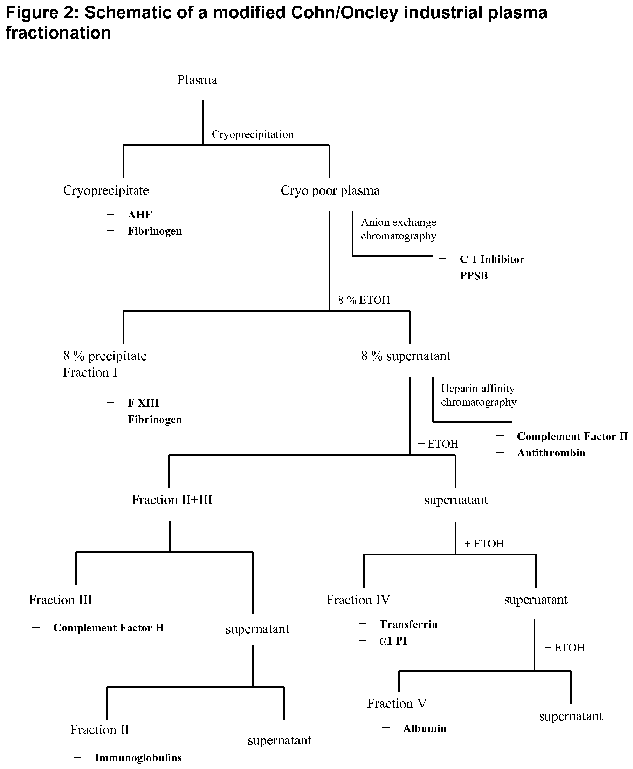

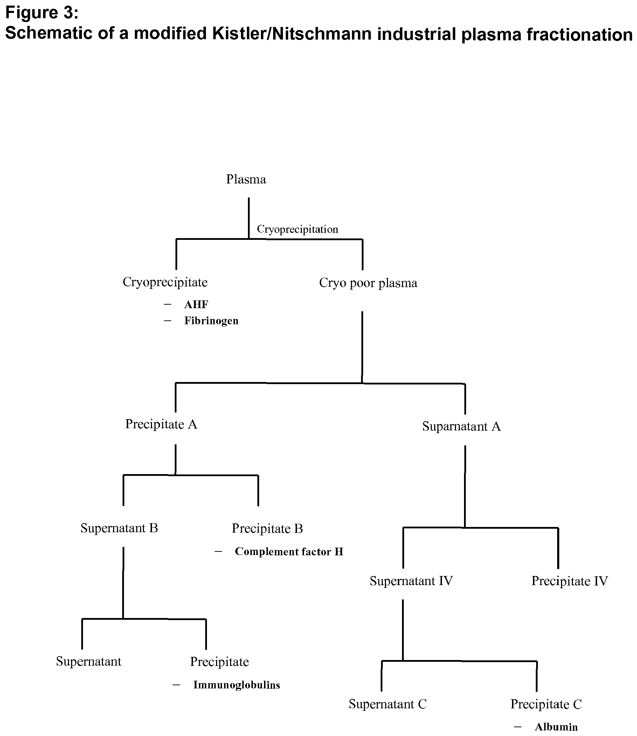

Human blood plasma is industrially utilized for decades for the manufacturing of widely established and accepted plasma-protein products such as e.g. human albumin, immunoglobulin preparations (IgG), clotting factor concentrates (clotting Factor VIII, clotting Factor IX, prothrombin complex etc.) and inhibitors (Antithrombin III, C1-inhibitor etc.). In the course of the development of such plasma-derived drugs, plasma fractionation methods have been established, leading to intermediate products enriched in certain protein fractions, which then serve as the starting material for the according plasma-protein product. Typical processes are reviewed e.g. in Schultze HE, Heremans JF; Molecular Biology of Human Proteins. Volume I: Nature and Metabolism of Extracellular Proteins 1966, Elsevier Publishing Company; p. 236-317 and simplified schematics of such processes are given in Figure 2 (Cohn/Oncley) and Figure 3 (Kistler Nitschmann). These kinds of separation technologies allow for the manufacturing of several therapeutic plasma-protein products from the same plasma donor pool being economically advantageous over producing only one plasma-protein product from one donor pool and have, therefore, being adopted as the industrial standard in blood plasma fractionation.

In a first step FVIII, von Willebrand factor and fibrinogen are precipitated from plasma (cryoprecipitation) and the remaining cryo poor plasma may be adsorbed to matrices to isolate proteins of the prothrombin complex (PT adsorption, PPSB) and or to adsorb C1-inhibitor (C1 adsorption). Usually this adsorption is done using anion-exchange (AEX) matrices like DEAE or QAE.

In the Cohn process then a precipitation at 8% ethanol is done which precipitates FXIII and more fibrinogen. The 8% ethanol supernatant is either subjected directly to further precipitation steps by adding the ethanol concentration ultimately leading to products like immunoglobulins, albumin, complement factor H, transferrin and alpha-I-plasma inhibitor.

Optionally the 8% supernatant may be additionally adsorbed to isolate antithrombin III (AT III adsorption). This step is usually done by using heparin or heparin-like substances.

Current manufacturing processes normally allow some flexibility such that not always all adsorptions are done depending on the relative demand for the different products. In the production of immunoglobulins and albumin there may be either:
1: No adsorption steps performed
2: PT adsorption step is solely performed
3: PT adsorption is followed by adsorption of C1 esterase inhibitor
4: PT adsorption is followed by ATIII adsorption
5: Complete adsorption process (adsorption of PT, C1 esterase inhibitor and ATIII)

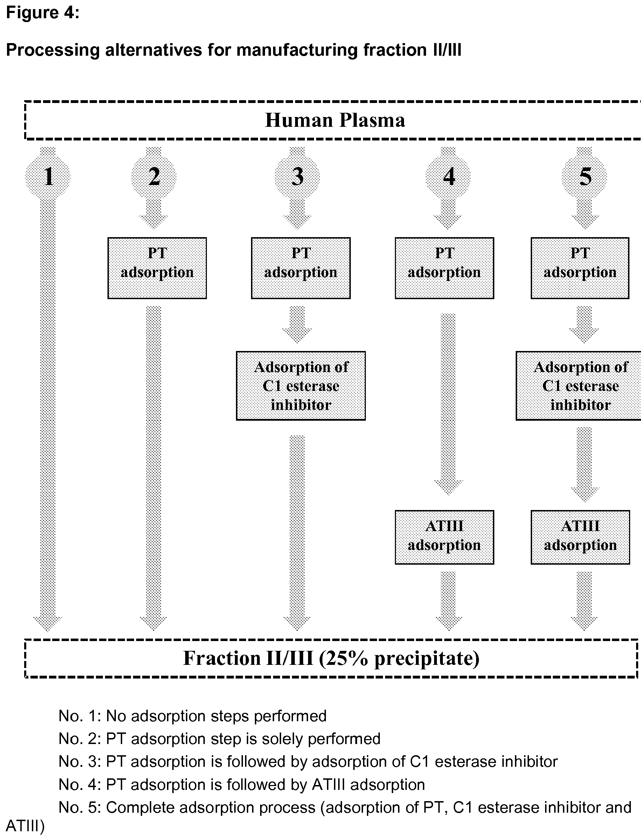

A graph depicting said alternative manufacturing methods is shown in Figure 4. A non limiting example of such a manufacturing process is described in Example 1.2. The scope of the invention is, however, not limited to pharmaceutical preparations comprising immunoglobulins as will become evident below.

It has now been found that especially after an adsorption with AEX matrices during the PT and the C1 adsorption activated serine proteases like kallikrein, FXIa or FXIIa could be detected in subsequent products. Surprisingly samples which in addition to an adsorption to an AEX matrix were also adsorbed to heparin or heparin-like substances showed significantly reduced levels of kallikrein and/or FXIa-like activity. This leads to a significantly decreased procoagulatory activity of the AT III adsorbed products. This reduced procoagulatory activity reduces the risk of adverse events when such product is administered to patients, thromboembolic events, skin reactions, bronchospasms, hypoxia, severe rigors, tachycardia, stomach aches and raised blood pressure.

Not wanting to be bound by theory this effect may be explained in that the AEX matrices activate FXII to FXIIa which in turn activates prekallikrein to kallikrein and FXI to FXIa. A further adsorption to remove C1 inhibitor may lead to further activation and also removes with C1 inhibitor an important inhibitor of kallikrein. When now these kallikrein FXIa and FXIIa containing samples come into contact with heparin or heparin-like matrices, AT III - which is still usually present at this stage of plasma protein processing - binds to the heparin or heparin-like matrix, is activated and subsequently inactivates FXIa and kallikrein by irreversibly binding to both proteins thereby removing these potential thrombogenic proteins. Therefore the invention will be applicable in any solution comprising plasma proteins which may contain activated serine proteases as long as the solution also comprises AT III.

AT III is a plasma protein and a serine proteinase inhibitor that inactivates thrombin and the other serine proteases responsible for the generation of thrombin. The anticoagulant activity of heparin or heparin-like substances derives from their ability to potentiate the inhibitory activity of AT III by mechanisms that are similar to the physiologic activation of ATIII by vessel wall heparin sulfate proteoglycans (HSPGs). AT III serves as an important regulator of hemostasis and thrombosis at several levels by blocking (a) thrombin-mediated fibrin clot formation, (b) common pathway factor Xa mediated thrombin generation, and (c) coagulation factors that are higher up in the intrinsic and extrinsic pathways (FIXa, FXIa, FXIIa and plasma kallikrein and FVIIa (Colman et al., Hemostasis and Thrombosis, 5th edition, 2006 Lippincott Williams, p. 235 f.).

Binding of AT III to heparin or heparin-like substance leads to a conformational change in AT III transforming the molecule into a highly active state which has a several thousand fold enhanced inhibitory activity to activated serine proteases like activated coagulation factors by forming irreversibly a covalent bond to the activated serine protease. Upon forming this covalent bond the serine protease loses irreversibly its biological function as a serine protease.

The invention is therefore about a method to reduce the activity of FXIa in an immunoglobulin pharmaceutical preparation derived from a plasma fraction said plasma fraction comprising antithrombin III, the method comprising contacting the plasma fraction with heparin thereby reducing the activity of FXIa per ml of the plasma fraction, wherein the heparin is covalently coupled to a matrix and the plasma fraction has been preadsorbed to an anion exchange matrix, wherein the anion exchange matrix is an anion exchange membrane.

A "heparin or heparin-like substance" in the sense of the invention is any form of heparin or heparin-related substance which cause when contacting AT III the activation of AT III, i.e. that AT III adapts the conformation which has a high affinity to form covalent complexes with activated serine protease, preferentially activated coagulation factors.

Heparin-like substances consist of a group of products derived from heparin, made by one or more chemical modifications. For example, sulfated heparin is a derivative in which all primary hydroxyls in glucosamine residues and a large proportion of secondary hydroxyl groups in disaccharide units have been substituted by O-sulfate esters; carboxyl reduced heparin is a derivative in which the carboxyl group of uronic acid residues of heparin have been reduced to alcohols; periodate-oxidized heparin is a derivative in which all unsulfated uronic acid residues of heparin are oxidized by periodic acid. Other heparin derivatives include, for example, de-O-sulfated heparin, 2-O-desulfated heparin, fully N-acetylated heparin, fully N-sulfated heparin, de-N-sulfated heparin, de-N-acetylated heparin. "Heparin or heparin-like substances" in the sense of the invention encompass unfractionated heparin, high-molecular weight heparins, low-molecular weight heparins and synthetic heparin analogues like fondaparinux.

"Heparin or heparin-like substances" may be used according to the invention by contacting a plasma fraction which comprises activated serine proteases, preferentially coagulation factors wherein the heparin or heparin-like substance is covalently coupled to a matrix. Here the covalent complex of AT III with the activated coagulation factor remains bound to the matrix. Alternatively the heparin or heparin-like substance may be added to a plasma fraction as a soluble substance. Then the covalent complex of AT III with the activated coagulation factor either precipitates or remains in solution.

"Reducing the specific activity of at least one activated serine protease per ml of the plasma fraction" in the sense of the invention means that the method of the invention leads to a decrease of the activity of at least one serine protease per volume of the plasma fraction which comprises antithrombin III. The reduction of the activity may be due only to the irreversible binding of the activated serine protease to the heparin-activated antithrombin III, when heparin or the heparin-like substance is added in solution whereas the antigen content of the activated serine protease does not change or may also lead to a reduction of the amount of the activated serine protease if the heparin or heparin-like substance is coupled to a matrix which is subsequently separated from the plasma fraction and where the serine protease remains covalently coupled to antithrombin III on the matrix.

An "adverse event" in the sense of the invention is any effect caused by the administration of the pharmaceutical preparation caused by activated serine proteases and may comprise thrombosis, skin reactions, bronchospasms, hypoxia, severe rigors, tachycardia, stomach aches and raised blood pressure.

"Plasma derived proteins" can comprise any protein which is isolated from human plasma after the 8% ethanol precipitation step according to Cohn or an equivalent step according to other methods for plasma fractionation. In an example "plasma derived proteins" mean all proteins which are isolated from human plasma where intermediates thereof have been contacted with an AEX matrix. "Plasma derived proteins" comprise for example immunoglobulins, albumin, complement factor H, alpha-I-proteinase inhibitor and transferrin.

A "plasma fraction" can be any plasma derived solution or redissolved precipitate where at least part of the proteins originate from human plasma.

Factor XI is a coagulation protein and a serine protease produced in the liver and circulates in plasma at approximately 5 µg/ml (30 nM). FXI consists of two identical 80 kDa subunits linked by disulfide bonds. Cleavage of FXI by activated factor XII or thrombin converts each subunit into a two-chain form and generates two active sites per FXIa molecule (Bagila FA, Seaman FS, Walsh PN. The apple 1 and 4 domains of factor XI act to synergistically promote the surface-mediated activation of factor XI by factor XIIa. Blood 1995; 85:2078). The activity of FXIa is regulated by platelets and by several proteinase inhibitors. Natural substrate for FXIa is solely FIX; the only cofactor required for this reaction are calcium ions.

Prekallikrein is a 88 kDa single chain glycoprotein produced in the liver. The plasma concentration of PK is 50 µg/ml (550 nM), approximately 75% of which circulates in complex with high molecular weight kininogen and the remainder as free PK (Hojima Y, Pierce JV, Pisano JJ. Purification and characterization of multiple forms of human plasma prekallikrein. J Biol Chem 1985; 260:400-406). Limited proteolysis of PK by FXIIa generates the active serine protease kallikrein (Dela Cadena R, Watchtfogel YT, Colman RW. Hemostasis and Thrombosis, 3rd edition 1994. pp. 219-240).

Factor XII (Hageman factor) is a 76 kDa, single chain glycoprotein produced in the liver. In plasma, FXII circulates as a protease zymogen at a concentration of approximately 30 µg/ml (400 nM). Upon vascular injury FXII binds to negatively charged extravascular surfaces which facilitate activation of the zymogen to the active serine protease (Pixley RA, Schapira M, Coleman RW. The regulation of human factor XIIa by plasma proteinase inhibitors. J Biol Chem 1985; 260(3):1723-1729). The activity of FXIIa in plasma is regulated predominantly by C1 inhibitor.

An "intermediate" of a pharmaceutical preparation comprising one or more plasma proteins can be any intermediate fraction during the purification of said one or more plasma proteins and comprises for example any supernatant from a precipitation step during the purification or any eluate of a matrix used for purification of a plasma derived protein.

The method of the invention is especially useful as the plasma fraction which is contacted with heparin is prior adsorbed to an anion-exchange matrix.

In the sense of the invention an "anion exchange matrix" refers to a solid phase which is positively charged at the time of protein binding, thus having one or more positively charged ligands attached thereto. Any positively charged ligand attached to a solid phase suitable to form the anionic exchange matrix can be used, such as quaternary amino groups. For example, a ligand used in AEC can be a quaternary ammonium, such as quaternary alkylamine and quaternary alky lalkanol amine, or amine, diethylamine, diethylaminopropyl, amino, trimethylammoniumethyl, trimethylbenzyl ammonium, dimethylethanolbenzyl ammonium, and polyamine. Alternatively, for AEC, a membrane having a positively charged ligand, such as a ligand described above, can be used instead of an anion exchange matrix.

Commercially available anion exchange matrices include, but are not limited to, DEAE cellulose, POROS® PI 20, PI 50, HQ 10, HQ 20, HQ 50, D 50 from Applied Biosystems, MonoQ®, MiniQ, Source™ 15Q and 30Q, Q, DEAE and ANX Sepharose® Fast Flow, Q Sepharose® high Performance, QAE SEPHADEX™ and FAST Q SEPHAROSE® from GE Healthcare, WP PEI, WP DEAM, WP QUAT from J.T. Baker, Hydrocell DEAE and Hydrocell QA from Biochrom Labs Inc., UNOsphere™ Q, Macro-Prep® DEAE and Macro-Prep® High Q from Biorad, Ceramic HyperD® Q, ceramic HyperD® DEAE, Q HyperZ®, Trisacryl® M and LS DEAE, Spherodex® LS DEAE, QMA Spherosil® LS, QMA Spherosil® M from Pall Technologies, DOWEX® Fine Mesh Strong Base Type I and Type II Anion Matrix and DOWEX® MONOSPHER E 77, weak base anion from Dow Liquid Separations, Matrex Cellufine A200, A500, Q500, and Q800, from Millipore, Fractogel® EMD TMAE3 Fractogel® EMD DEAE and Fractogel® EMD DMAE from EMD, Amberlite™ weak and strong anion exchangers type I and II, DOWEX® weak and strong anion exchangers type I and II, Diaion weak and strong anion exchangers type I and II, Duolite® from Sigma- Aldrich, TSK gel® Q and DEAE 5PW and 5PW-HR, Toyopearl® SuperQ-650S, 650M and 650C3 QAE-550C and 650S, DEAE- 650M and 650C from Tosoh, and QA52, DE23, DE32, DE51, DE52, DE53, Express-Ion™ D and Express-Ion™ Q from Whatman.

The AEX matrix is an anion exchange membrane. Commercially available anion exchange membranes include, but are not limited to, Sartobind® Q from Sartorius, Mustang® Q from Pall Technologies and Intercept™ Q membrane from Millipore.

### Figures

- **Figure 1:**: Coagulation cascade
- **Figure 2:**: Schematic of a modified Cohn/Oncley industrial plasma fractionation
- **Figure 3:**: Schematic of a modified Kistler/Nitschmann industrial plasma fractionation
- **Figure 4:**: Processing alternatives for manufacturing fraction II/III
- **Figure 5:**: Analytical Results (Predicted Response Graph)
The statistical analysis of testing results was performed by using the computer program Cornerstone Version 5.0 (Applied Materials Co.).
- **Figure 6:**: Correlation between Prekallikrein-Ag and Kallikrein-like activity (a); correlation between Factor XI-Ag and Factor XI-like activity (b)

### EXAMPLES

### Example 1:

### Example 1.1 - Introduction

An analytical investigation of an immunoglobulin for subcutaneous administration (SC Immunoglobulin) was performed. Various analytical methods were applied with regard to the potential presence of trace amounts of activated clotting factors and proteolytic activity in the SC Immunoglobulin.

The evaluation of analytical data revealed that the SC Immunoglobulin batches contain levels of procoagulant activity in correlation to applied variations of the adsorption scheme. A comparison of adsorption schemes of individual batches revealed that higher levels of procoagulant activity are correlated to high PT and low ATIII adsorption levels during the plasma fractionation process steps.

Based on the finding of procoagulant activity the production process was adapted to include maximum ATIII adsorption. The subsequent examples provide strong evidence that a high level of ATIII adsorption leads to a significant decrease in the procoagulant activity of the SC Immunoglobulin product.

### Example 1.2 - Manufacturing Process for a SC Immunoglobulin

The drug substance was prepared by a modified Cohn Fractionation (Cohn EJ, Strong LE, et al. Preparation and properties of serum and plasma proteins; a system for the separation into fractions of the protein and lipoprotein components of biological tissues and fluids. J Am Chem Soc 1946; 68:459-75). Plasma was thawed, the formed cryoprecipitate was separated and contained fibrinogen and antihemophilic Factor VIII / von Willebrand factor complex. With the supernatant, cryodepleted plasma, optional batch adsorption of the prothrombin complex and C1 esterase inhibitor could be optionally performed (see Figure 4). Subsequently, ethanol was added to the cryodepleted plasma or filtrate from previous adsorption(s) to adjust an ethanol concentration of 8%. The precipitate, Cohn Fraction I, mainly contained fibrinogen and factor XIII and was separated by filtration. With the supernatant an optional batch adsorption of antithrombin III could be performed.

60 to 100 mL heparin affinity resin per liter cryodepleted plasma are suspended with the same quantity of Fraction I supernatant in a chromatography column. The obtained suspension is added to the residual quantity of the batch for fractionation. The pH value is adjusted to 6.5 (±0.1) with hydrochloric acid while stirring. The total stirring time is 45 to 60 min at a product temperature of 0 (±2) °C. Subsequently, the product solution is filtered through a filter bag and the filtrate is transferred for further plasma fractionation.

The Fraction I supernatant or flow through fraction from previous ATIII adsorption was precipitated at an ethanol concentration of 25%. The resulting precipitate, Cohn Fraction II/III, was obtained by centrifugation and contained mainly immunoglobulins. Fraction II/III is frozen and stored at -20°C or below.

After dissolution in an aqueous glycine solution the fraction II/III was further precipitated at 10% ethanol concentration in the presence of 0.5% fatty alcohol (also referred to as 10% pre-precipitation because it precedes the main 20% precipitation). The precipitate containing mainly IgM, IgA and lipoproteins was removed by filtration.

The supernatant was further precipitated at an ethanol concentration of 20%. The formed precipitate which consisted mainly of IgG (Gammaglobulin paste) was obtained by filtration. Crude Gammaglobulin paste was frozen. Afterwards, it was dissolved and subjected to adsorption by using an ion exchange resin and activated carbon to remove residual albumin and fatty alcohol. Impurities bound to the resin and activated carbon were removed by filtration, respectively. The filtrate was subsequently stabilized with sucrose and glycine. The stabilized solution was pasteurized as an effective virus reduction step. After completion of pasteurization, the stabilizers were removed by ultrafiltration (dialysis). The solution was then concentrated to obtain the drug substance, the immunoglobulin ultraconcentrate.

After the pooling process of the immunoglobulin ultraconcentrate lots, bulk adjustment was performed and the adjusted bulk solution was then filtered through clarification cartridge filters followed by sterilizing filtration. Immediately after completion of the filling process, vials were automatically stoppered and sealed with crimp caps.

### EXAMPLE 1.2 - ANALYTICAL METHODS

### 1.2.1 Factor XIa-like activity (aPTT approach in FXI depleted plasma)

The activated partial thromboplastin time (aPTT) is a coagulation test that encompasses all steps of the intrinsic pathway of blood coagulation from the activation of the contact phase system to fibrin formation. During the preincubation phase of the aPTT assay, Factor XII was activated by negatively charged surfaces (e.g. Pathromtin SL) and activated Factor XI to Factor XIa in the presence of high molecular weight kininogen. The result of this initial step was to produce FXIa. The clot measurement phase of the aPTT assay took place after recalcification during which FXIa activated FIX, thus continuing the cascade through FXa to thrombin.

Factor XI-deficient plasma was applied and the presence of activated coagulation factor XI in the sample especially led to a decrease in the coagulation time. The sample was considered as 'activated' with lower clotting times caused by FXIa-like activity in the sample. A longer clotting time indicated a lower procoagulant acivity.

Factor XI-deficient plasma and Pathromtin SL reagent were incubated for 6 minutes at +37°C. Pathromtin SL is a reagent consisting of phospholipid and a surface activator (silicon dioxide particles) used to activate the factors of the intrinsic coagulation system. Subsequently, a sample was added, together with 25 mM CaCl2 solution, which triggers the coagulation process. The time between CaCl2 addition and clot formation was measured. Buffer was used as control sample and as diluent for product sample preparation. The buffer used for FXIa testing experiments consisted of purchased imidazole buffer and 1 % human albumin. Factor XIa reference material was used for quantification purposes and the test data were presented as FXIa equivalence.

### 1.2.2 Kallikrein-like activity (chromogenic substrate S-2302)

Kallikrein-like activity was estimated by means of the cleavage of the chromogenic substrate H-D-Pro- Phe-Arg-pNA (chromogenic substrate S-2302, Chromogenix Co.) and absorbance measuring of pNA at 405 nm. S-2302 is a chromogenic substrate which mainly reacts with plasma kallikrein, and therefore is used for the determination of kallikrein-like activity.

After addition of the chromogenic substrate solution, the samples were incubated at +37°C for 30 minutes. The active kallikrein in the sample is able to cleave the substrate in a concentration dependent manner. This led to a difference in absorbance (optical density) between the pNA formed and the original substrate which was measured photometrically at 405 nm. Moreover, the evaluation was performed on the basis of a standard curve by applying commercial standard reference material of kallikrein.

### 1.2.3 Proteolytic activitiy (chromogenic substrates)

The colorimetric determination of proteolytic activity in samples was performed by applying chromogenic substrates. After addition of the chromogenic substrate solution, the samples (1:20 diluted) were incubated at +37°C for 30 minutes. Proteolytic activity in the sample is able to cleave the substrate in a concentration dependent manner. The method for the determination of activity is based on the difference in absorbance (optical density) between the pNA formed and the original substrate. The rate of pNA formation, i.e. the increase in absorbance per second at 405 nm, is proportional to the enzymatic activity and was determined.

The following table (Table 1) provides an overview of the substrates applied within this study and the respective specificity.

**Table 1a: Overview of chromogenic substrates applied**

| Label (Chromogenix Co.) | Chromogenic substrate mainly for* |
|---|---|
| S-2302 | Kallikrein-like activity |
| S-2366 | Activated protein C, FXIa |
| S-2238 | Thrombin |
| S-2765 | FXa |
| S-2251 | Plasmin, streptokinase-activated plasminogen |
| S-2288 | Broad spectrum of serine proteases, several proteases with arginine specifity |

| | |
|---|---|
| * according to Chromogenix Co., Italy | |

### 1.2.4 Factor XI ELISA

Human FXI antigen in SC Immunoglobulin samples was quantitatively determined by using commercially available paired antibodies (sandwich-style ELISA), e.g. supplied by Coachrom Diagnostika Co. A polyclonal antibody to FXI was coated onto wells of a microtitre plate and captures FXI which is obtainable in the sample or in the standard reference solution. Afterwards, a horseradish peroxidase conjugated antibody to FXI (polyclonal) was added to the well aiming to bind to the captured FXI. After removal of unbound antibodies by several washing steps, a peroxidase reactive substrate solution was added which leads to a coloration in a concentration dependent manner.

The coloration was formed in proportion to the amount of FXI present in the sample. This reaction was terminated by the addition of acid and is measured photometrically at 450 nm by utilizing BEPII or BEPIII systems (Siemens Co.). Moreover, a standard curve was applied by using standard human plasma (Siemens Co.).

Human FXI was detected as well as human FXIa due to the cross-reactivity of both with the polyclonal paired antibodies applied.

### 1.2.5 Prekallikrein ELISA

Human prekallikrein antigen in SC Immunoglobulin samples was quantitatively determined by using commercially available paired antibodies (sandwich-style ELISA) supplied by Affinity Biologicals Co. A polyclonal antibody to prekallikrein is coated onto wells of a microtitre plate and captures prekallikrein which is obtainable in the sample or in the standard reference solution. Afterwards, a horseradish peroxidase conjugated antibody to prekallikrein (polyclonal) was added to the well aiming to bind to the captured prekallikrein. After removal of unbound antibodies by several washing steps, a peroxidase reactive substrate solution was added which led to a coloration in a concentration dependent manner.

The coloration was formed in proportion to the amount of prekallikrein present in the sample. This reaction was terminated by the addition of acid and the color produced quantified by photometric measurement at 450 nm. BEPII or BEPIII systems (Siemens Co.) were used for the determination.

Human prekallikrein was detected as well as human kallikrein due to the cross-reactivity of both with the polyclonal paired antibodies applied.

### 1.2.6 Factor XII ELISA

Human FXII antigen in SC Immunoglobulin samples was quantitatively determined by using commercially available paired antibodies (sandwich-style ELISA), e.g. supplied by Kordia Co. The test approach applied is comparable to the determination of FXI and PK by ELISA technology as mentioned above.

### 1.3 Test results

29 lots of SC Immunoglobulin drug product manufactured at CSL Behring Marburg (Germany) were analyzed for procoagulant activity. The lots were chosen on the basis of their adsorption scheme. The listed percentage of the adsorption rate per SC Immunoglobulin lot is the result of mixing various fraction II/III intermediate lots with different adsorption levels.

For example, the total amount (100%) of fraction II/III pastes used for SC Immunoglobulin sample no. 13 was PT adsorbed, whereas 59.8% passed the C1 esterase inhibitor adsorption step and 1.8% the ATIII adsorption.

Supplementary testing activities and analyses for SC Immunoglobulin with regard to the potential presence of trace amounts of activated clotting factors and proteolytic activity in SC Immunoglobulin drug product were also initiated. For the identification and quantification of residual clotting factors several complementary approaches were performed:
- Trace amounts of FXI and FXIa were measured by a modified aPTT test performed with FXI-deficient plasma.
- Kallikrein-like activity was measured by applying the chromogenic substrate S-2302 (Chromogenix Co.) due to being generally supposed as major impurities of immunoglobulin preparations.
- The potential presence of proteolytic activity was investigated by using chromogenic substrates characterizing a wide range of proteases.
- ELISA technology was used for the determination of FXI-, PK- and FXII-antigen, respectively.

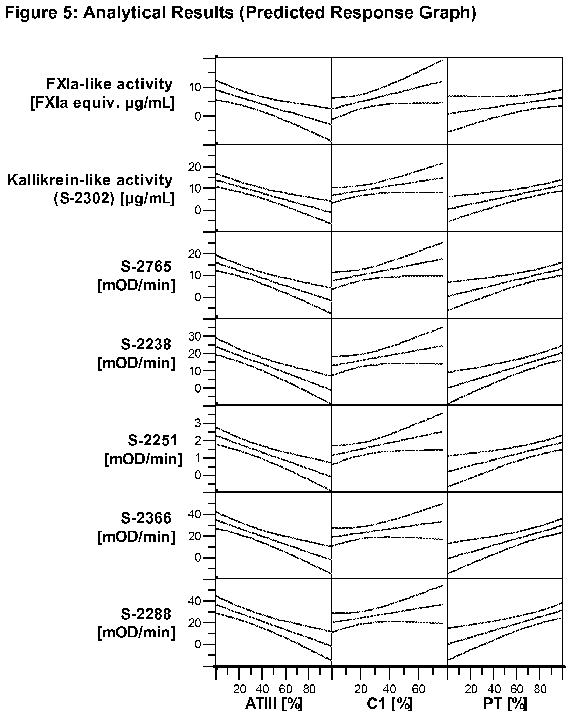

The results of SC Immunoglobulin drug product investigated by FXIa-like activity, Kallikrein-like activity and proteolytic activity are summarized in Figure 5. The statistical analysis of testing results was performed by using the computer program Cornerstone Version 5.0 (Applied Materials Co.). The evaluation comprises 29 lots of SC Immunoglobulin in total.

**Table 1b: Selected lots of SC Immunoglobulin drug product for further analytical evaluation**

| **Sample no.** | **Adsorption scheme** | | | **FXIa**-**like activity** | **Kallikrein-like activity (S-2302) [µg/mL]** |
|---|---|---|---|---|---|
| | **[%]** | | | **FXIa equiv. [µg/mL]** | |
| | **PT** | **C1** | **ATIII** | | |
| *Lots without any adsorption steps:* | | | | | |
| 1 | 0 | 0 | 0 | 0.06 | < 0.8 |
| 2 | | | | 0.03 | < 0.8 |
| 3 | | | | 0.18 | < 0.8 |
| 4 | | | | 0.08 | < 0.8 |

| *Lots with both 100% PT and ATIII adsorption, but differing amounts of C1 adsorbed material:* | | | | | |
|---|---|---|---|---|---|
| 5 | 100 | 41.3 | 100 | < 0.01 | < 0.8 |
| 6 | | 43.4 | | < 0.01 | < 0.8 |
| 7 | | 60.5 | | < 0.01 | < 0.8 |

| *Lots with 100% PT and without almost any ATIII adsorption, but differing amounts of C1 adsorbed material:* | | | | | |
|---|---|---|---|---|---|
| 8 | 100 | 0 | 0 | 14.14 | 20.0 |
| 9 | | 8.8 | 0 | 6.56 | 11.9 |
| 10 | | 13.1 | 0 | 11.10 | 15.9 |
| 11 | | 30.8 | 0 | 12.90 | 19.5 |
| 12 | | 34.8 | 0 | 17.96 | 18.8 |
| 13 | | 59.8 | 1.8 | 23.98 | 23.7 |

| *Lots with 100% PT and 70 to 80% C1 adsorbed material, but differing amounts of ATIII adsorbed material:* | | | | | |
|---|---|---|---|---|---|
| 14 | 100 | 76.4 | 15.1 | 14.94 | 21.6 |
| 15 | | 72.3 | 59.2 | 3.73 | 12.3 |

| *Additional lots randomly chosen:* | | | | | |
|---|---|---|---|---|---|
| 16 | 41.1 | 41.1 | 63.2 | 0.13 | < 0.8 |
| 17 | 4.6 | 0 | 86.4 | < 0.01 | < 0.8 |
| 18 | 77.7 | 25.0 | 100 | < 0.01 | < 0.8 |
| 19 | 86.5 | 13.5 | 0.3 | 2.13 | 6.0 |
| 20 | 77.4 | 0 | 30.3 | 1.51 | 3.0 |
| 21 | 100 | 6.2 | 55.0 | 0.33 | 5.0 |
| 22 | | 3.3 | 11.9 | 3.42 | 9.8 |
| 23 | | 26.8 | 29.6 | 5.66 | 11.3 |
| 24 | | 14.1 | 58.4 | 2.67 | 7.6 |
| 25 | | 0.9 | 1.8 | 8.11 | 14.4 |
| 26 | | 1.9 | 4.0 | 5.40 | 13.4 |
| 27 | | 30.7 | 40.4 | 8.19 | 10.1 |
| 28 | | 17.6 | 22.6 | 5.57 | 15.9 |
| 29 | | 20.4 | 10.1 | 3.71 | 17.5 |

SC Immunoglobulin sample no. 13 was selected as a batch with a high level of procoagulant activity whereas sample no. 7 represents SC Immunoglobulin drug product with a low level of procoagulant activity concerning all analytical methods applied. Both lots were compared and the test results are shown in Table 3. Both batches differ in the manufacturing process of fraction II/III (25% precipitate) used as starting intermediate fraction for the further manufacturing process of the respective SC Immunoglobulin drug product. The total amount (100%) of fraction II/III pastes used for both batches was PT adsorbed and about 60% passed the C1 esterase inhibitor adsorption in both cases. However, 100% of fraction II/III used for sample no. 7 was ATIII adsorbed whereas only an insignificant amount of fraction II/III passed the ATIII adsorption step (1.8 %) which was subsequently used for sample no. 13.

The data demonstrate that drug product with a high ATIII adsorption rate in the process (sample no. 7) contains very low levels of activated clotting factors and proteolytic activity in the drug product (see Table 3) in comparison to product manufactured with very little ATIII adsorption (sample no. 13).

The method used for the determination of proteolytic activity in SC Immunoglobulin drug product was performed by applying chromogenic substrates (S-2765, S-2238, S-2251 and S-2288) and indicated a significantly lower effect in the drug product if a ATIII adsorption step was subsequently performed. Due to a relatively low reaction by using substrate S-2251, the presence of plasmin seems to be less relevant for SC Immunoglobulin drug product. Moreover, an increased depletion of FXI-Ag (factor of 3.2), PK-Ag (factor of 6.6) and FXII-Ag (factor of 1.2) measured by ELISA was determined and correlated to the processed ATIII adsorption on a high level. The above data were supported by analytical results of intermediate fractions obtained before and after thethe ATIII adsorption step ("Fraction I supernatant prior to ATIII adsorption" vs. "After the ATIII adsorption step") as presented in the following table, which shows a significant decrease in FXIa-like activity, as well as FXI, FXII and PK antigen content.

**Table 2: Comparison of intermediate fractions prior and after the AT III adsorption step**

| Intermediate fraction | ATIII content | FXI-ELISA | FXIa-like activity | FXII-ELISA | PK-ELISA |
|---|---|---|---|---|---|
| | [IU/mL] | [µg/mL] | [ng/mL] | [mlU/mL] | [µg/mL] |
| Fraction I supernatant prior to ATIII adsorption | 0.7 | 3.4 | 295 | 601 | 6.3 |
| After the ATIII adsorption step | 0.1 | 0.3 | <10 | 16 | 4.5 |

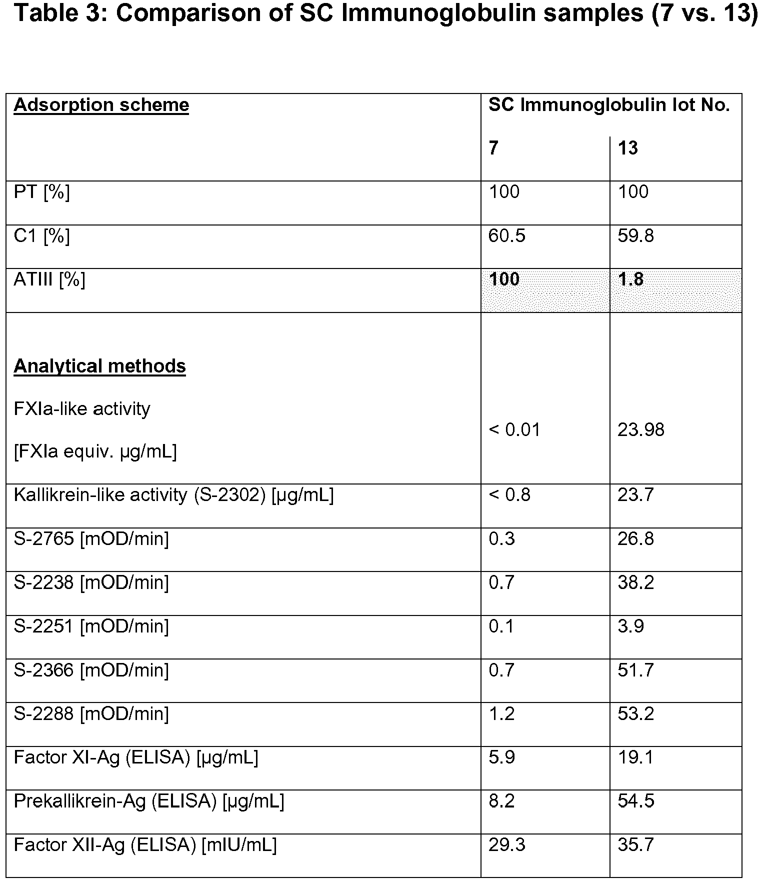

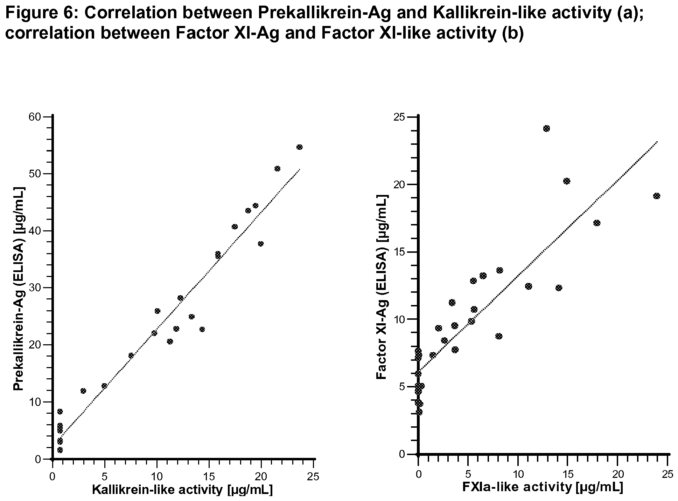

The test results revealed that a high level of ATIII adsorption leads to a significant decrease in the procoagulant activity of SC Immunoglobulin drug product. To further detail the effect of the ATIII adsorption the content of specific clotting factors in drug product was measured by ELISA. The strong correlation between both prekallikrein-Ag and kallikrein-like activity and FXI-Ag and FXIa-like activity is shown in Figure 6.

Increasing ATIII adsorption led to a depletion of FXI, PK and FXII measured as antigen by ELISA as well as a reduction of FXIa- and kallikrein-like activity as shown in Table 2, Table 3 and Figure 5.

The analysis revealed that SC Immunoglobulin lots manufactured with high level of ATIII adsorption exhibit low procoagulant activity. These lots reveal lower concentrations of FXI-like activity (in FXI-depleted plasma) as well as a lower kallikrein-like activity values (PKA blank value). The determination of proteolytic activity in SC Immunoglobulin drug product via applying various chromogenic substrates (S-2765, S-2238, S-2251 and S-2288) indicated a significantly lower proteolytic activity in the drug product when ATIII adsorption level is high.

Increasing ATIII adsorption led to a depletion of FXI, PK and FXII measured as antigen by ELISA as well as a reduction of FXIa- and kallikrein-like activity as shown in Figure 5.

A strong correlation between both prekallikrein-Ag and kallikrein-Ag and FXI-Ag and FXIa-like activity was shown. It was shown that procoagulant activity detected in SC Immunoglobulin is mainly caused by the content of kallikrein and FXIa. The data generated within this study provide strong evidence that a high level of ATIII adsorption leads to a significant decrease in the procoagulant activity of SC Immunoglobulin drug product.

## Claims

1. Method to reduce the activity of FXIa in an immunoglobulin pharmaceutical preparation derived from a plasma fraction said plasma fraction comprising antithrombin III, the method comprising contacting the plasma fraction with heparin thereby reducing the activity of FXIa per ml of the plasma fraction, wherein the heparin is covalently coupled to a matrix and the plasma fraction has been preadsorbed to an anion exchange matrix, wherein the anion exchange matrix is an anion exchange membrane.

2. The method of claim 1, wherein the heparin covalently coupled to a matrix is subsequently separated from the plasma fraction.

## Patentansprüche

1. Verfahren zur Verringerung der Aktivität von FXIa in einer pharmazeutischen Immunglobulinzubereitung, die von einer Plasmafraktion abgeleitet ist, wobei die Plasmafraktion Antithrombin III umfasst, wobei das Verfahren Inkontaktbringen der Plasmafraktion mit Heparin umfasst, wodurch die Aktivität von FXIa pro ml Plasmafraktion verringert wird, wobei das Heparin kovalent an eine Matrix gekoppelt ist und die Plasmafraktion an eine Anionenaustauschermatrix voradsorbiert wurde, wobei es sich bei der Anionenaustauschermatrix um eine Anionenaustauschermembran handelt.

2. Verfahren nach Anspruch 1, wobei das an eine Matrix kovalent gekoppelte Heparin anschließend von der Plasmafraktion getrennt wird.

## Revendications

1. Méthode de réduction de l'activité de FXIa dans une préparation pharmaceutique d'immunoglobuline dérivée d'une fraction de plasma, ladite fraction de plasma comprenant de l'antithrombine III, la méthode comprenant la mise en contact de la fraction de plasma avec de l'héparine, réduisant ainsi l'activité de FXIa par ml de la fraction de plasma, où l'héparine est couplée de façon covalente à une matrice et la fraction de plasma a été pré-absorbée sur une matrice échangeuse d'anions, où la matrice échangeuse d'anions est une membrane échangeuse d'anions.

2. Méthode selon la revendication 1, où l'héparine couplée de façon covalente à une matrice est ensuite séparée de la fraction de plasma.
